# EUROPEAN PATENT APPLICATION

(11) **EP 4 837 583 A1**
(43) Date of publication of application: **11.11.2026**
(21) Application number: 25174677.2
(22) Date of filing: 07.05.2025
(51) Int. Cl.: A61K 31/7048, A61K 9/00, A61P 17/00, A61K 8/49, A61Q 19/00

(54) **PAEONIFLORIN USE FOR ALLEVIATION AND PROPHYLAXIS OF BACTERIA- AND MMP-INDUCED COLLAGEN DESTRUCTION OF SKIN AND GUM DERMIS**

(71) Applicant: SkyLab AG, 1066 Epalinges (CH)
(72) Inventor: Belous, Elena, 121309 MOSCOW (RU); Yarovaya, Alina Olegovna, 109518 MOSCOW (RU); Filatov, Viktor Andreevich, 119415 MOSCOW (RU)
(74) Representative: GLAWE DELFS MOLL

(57) **Abstract**

The invention relates to a composition formulated for application on and/or to a subject's connective soft tissue, wherein said composition comprises paeoniflorin. Said composition promotes fibroblast-mediated type I collagen synthesis in said connective soft tissue of said subject.

## Description

### FIELD OF INVENTION

The invention is directed to paeoniflorin, perfumery and cosmetic products comprising paeoniflorin, and the use thereof to take care of soft tissues and epithelial cells to alleviate the irritation of soft tissues, mucosa and epithelial integuments resulting from destruction of type I collagen, which is caused by the virulent agent invasion, ageing, alcohol and tobacco consumption, and exposure to UV radiation. The invention is related to paeoniflorin of plant origin, which can be found in roots, rhizomes, leaves, herbs and/or flowers, with the component level expressed as the active ingredient is 0.001 wt% to 0.08 wt%.

Paeoniflorin is intended to be used as the ingredient of pharmaceutical products and perfumery and cosmetic products to take care of soft tissues, mucosa, and epidermal cell, the use of paeoniflorin allows to alleviate the irritation due to promotion of type I collagen production in the young keratinocytes of skin epidermis, mucosal epithelium, secretory epithelium, nasal mucosa epithelium, gingival area, derma, scalp, and skin sebocytes. This component of natural origin is biodegradable, safe for the sensitive skin, and can be used in various care products applied on skin epidermis and mucosa, such as body lotions, facial cleansers, face, body, and hand care creams, facial serums, toothpastes, foam rinses, teething gels, gingival gels, tongue gels, stomatitis gels, tooth foams, ointments and creams for nasal mucosa, liquid shower gels, scalp shampoos, antidandruff shampoos and shampoos for seborrheic dermatitis and xerosis. The use of paeoniflorin prevents active tissue damage resulting from type I collagen degradation caused by bacterial invasion to skin tissues and oral mucosa, and thus reduces hypersensitivity and pain sensitivity for the prevention diseases affecting mucosa and epidermis, and alleviation of disease-associated conditions through stimulation of type I collagen production in tissues and reduction in number of TRPV1 channels.

### BACKGROUND OF THE INVENTION

Soft tissue is a general term designating various cell groups in the human organism. All tissues except bones or organs fall under the soft tissues. The soft tissues can be subdivided into connective tissues such as tendons or skin, fibrous tissues, fat, and synovial membranes, and non-connective tissues such as muscles, nerves and blood vessels. Key physiological functions of soft tissues are to attach, support and surround the organs and other body structures [Li, W. Damage Models for Soft Tissues: A Survey. J. Med. Biol. Eng. 36, 285-307 (2016). https://doi.org/10.1007/s40846-016-0132-1].

The skin is the largest organ of the body, which accounts for about 15% of total body weight in adults. The skin performs numerous vital functions including protection from external physical, chemical and biological factors and prevention of excessive water loss from the body, and is involved in temperature regulation. The skin is divided into three layers: epidermis, derma, and subcutaneous fat. The derma is the main part of the skin and is responsible for flexibility and elasticity, it protects the body from mechanical damage, binds water and is involved in thermoregulation. The derma together with epidermis are responsible for skin repair and remodeling in the course of wound healing [Kolarsick, Paul A. J. BS; Kolarsick, Maria Ann MSN, ARHP-C; Goodwin, Carolyn APRN-BC, FNP. Anatomy and Physiology of the Skin. Journal of the Dermatology Nurses' Association 3(4):p 203-213, July 2011. | DOI: 10.1097/JDN.0b013e3182274a98]. The extracellular matrix composed of collagen, elastic and reticulin fibers forms a base for dermal connective tissue [Harmansa, S., Erlich, A., Eloy, C., Zurlo, G., & Lecuit, T. (2023). Growth anisotropy of the extracellular matrix shapes a developing organ. Nature Communications, 14(1), 1220. doi:10.1038/s41467-023-36739-y].

Collagens I, III and V are the main components of extracellular matrix (ECM). ECM is a non-cellular component of all tissues and organs, which is a structural basis capable to direct the cell adhesion and migration and to control the cell growth and metabolism. The extracellular matrix supports the skin structure contributing to skin firmness and elasticity and resistance to damage. The collagens influencing the migration of Langerhans cells and epidermal T lymphocytes play an important role in the skin's immune response. Type I collagen accounts for about 80-90% of total skin collagen. The integrity of type I collagen fibrils, which are the essential structural component, provides for the skin mechanical strength. Disturbance of type I collagen synthesis leads to lax skin, which is prone to irritation, and to gravity-dependent ptosis of facial tissues. Reduced synthesis of collagens I and III associated with activation of matrix metalloproteases leads to the skin ageing. The studies show low levels of type I collagen expression and synthesis in the patents with atopic dermatitis eczema, psoriasis or acne. [Mempel M, Schmidt T, Boeck K, Brockow K, Stachowitz S, Fesq H, Schäfer T, Thomsen S, Schnopp C, Ring J, Probst R, Luppa P, Abeck D. Changes in type I collagen and type III collagen metabolism in patients with generalized atopic eczema undergoing medium-dose ultraviolet A1 phototherapy. Br J Dermatol. 2000 Mar;142(3):473-80. doi: 10.1046/j.1365-2133.2000.03359.x.], [Alexandre Mezentsev, Alexander Nikolaev, Sergey Bruskin, Matrix metalloproteinases and their role in psoriasis, Gene, Volume 540. Issue 1,2014, https://doi.org/10.1016/j.gene.2014.01.068], [Tsaousi A., Witte E., Witte K., Röwert-Huber H. J., Volk H. D., Sterry W., Wolk K., Schneider-Burrus S., and Sabat R., MMP8 is increased in lesions and blood of acne inversa patients: a potential link to skin destruction and metabolic alterations, Mediators of Inflammation. (2016) 2016, 8, 4097574, https://doi.org/10.1155/2016/4097574,]. This leads to the basal membrane thinning and consequently affects the integrity of the skin barrier and increases the skin permeability resulting in aggravation of inflammation and pruritus [Szalus, K., & Trzeciak, M. (2024). The Role of Collagens in Atopic Dermatitis. International Journal of Molecular Sciences, 25(14), 7647].

The gingival connective tissue is also made of collagen fibers, cells, and extracellular matrix. Collagens are responsible for the maintenance of dentine structure: they form a three-dimensional collagen network made mainly of type I collagen with a low level of collagens III and V, with hydroxyapatite crystals embedded into said network. In periodontium tissues type I collagen is the most abundant in periodontal ligament, dental pulp, and dentine, and the principal task of collagen is to provide the teeth attachment. Destruction or deficiency of type I collagen leads to weakened gum attachment to periosteum resulting in formation of periodontal pockets. The alveolar bone loss may progress and result in tooth falling out [Ines Kapferer-Seebacher, Elizabeth Oakley-Hannibal, Ulrike Lepperdinger, Diana Johnson, Neeti Ghali, Angela F. Brady, Glenda Sobey, Johannes Zschocke, Fleur S. van Dijk, Prospective clinical investigations of children with periodontal Ehlers-Danlos syndrome identify generalized lack of attached gingiva as a pathognomonic feature, Genetics in Medicine, Volume 23, Issue 2, 2021]. In addition, the disturbance of type I collagen synthesis, folding, and denaturation was found in pathogenesis of various periodontal diseases, such as parodontitis and periodontitis [Nakamura T, Yamashita M, Ikegami K, Suzuki M, Yanagita M, Kitagaki J, Kitamura M, Murakami S. Autophagy facilitates type I collagen synthesis in periodontal ligament cells. Sci Rep. 2021 Jan 14;11(1):1291].

Bacterial invasion with release of LPS capable to induce a wide range of pro-inflammatory reactions is one of the factors of type 1 collagen degradation in skin. In particular, LPS produced by *E. coli* can stimulate the macrophages and monocytes to produce proinflammatory cytokines, such as IL-1b, TNF-a, IL-6, IFN-γ, IL-12, and IFN-induced protein-10 [Madianos, P. N., Bobetsis, Y. A., & Kinane, D. F. (2005). Generation of inflammatory stimuli: how bacteria set up inflammatory responses in the gingiva. Journal of Clinical Periodontology, 32(s6), 57-71. doi:10.1111/j.1600-051x.2005.00821.x ]. As a response the skin produces a wide range of cytokines (IL-1, TNF-α, IL-6, G-CSF, TGF-β, and IL-10), chemokines (CXCL-8, IP-10), growth factors (IL-6, GM-CSF, and TGF-α) and other compounds; this results either in direct pathogen neutralization or in indirect activation of the other immune signals to trigger specific immune response [Abdallah, Florence, Mijouin, Lily, Pichon, Chantal, Skin Immune Landscape: Inside and Outside the Organism, Mediators of Inflammation, 2017, 5095293, 17 pages, 2017].

Degradation of type I collagen fibers is a process observed in pathogenesis of many skin diseases, such as eczema, acne, psoriasis, and atopic dermatitis. In psoriasis the elevation of TLR-7 activity resulting in production of proinflammatory cytokines IL-23 and IL-17 (IL-17A, IL-17B, IL-17C, IL-17D, and IL-17F) in the immune T-cells is a key inflammation mechanism. It was shown that IL-17A inhibits type I collagen expression in the skin cells [Yamamoto, T. (2020). Psoriasis and Connective Tissue Diseases. International Journal of Molecular Sciences, 21(16), 5803]. Proteogenomic analysis also showed suppressed expression of type I and IV collagens in skin affected by psoriasis, associated with impaired mechanical stability of the basal membrane, damaged keratinocytes, and dry skin [Kruglikov, I. L., & Wollina, U. (2017). Local effects of adipose tissue in psoriasis and psoriatic arthritis. Psoriasis: Targets and Therapy, 7, 17-25].

The skin contact with the bacterium *C*. *acnes* results in induction of innate immunity by means of Toll-like receptor 2 signaling pathway with the stimulation of pro-inflammatory gene expression, such as TNF-a, IL-6, IL-12, and CXCL8. This inflammatory medium promotes the MMP-9 synthesis in fibroblasts and endothelial cells leading to collagen break down. In addition, *C. acnes* causes the inflammatory response by initiation of Th17 and Th1 infiltration with the increased of IL-17 production in perifollicular areas. As was already mentioned, interleukins belonging to this family can down-regulate type 1 collagen production in the skin cells. The change in skin collagen level affects both the course and the consequences of the disease. Altered collagen I/collagen III ratio can lead to abnormal structure of extracellular matrix thus deteriorating the skin firmness and contributing to formation of hypertrophic acne scars [J. Moon, J.Y. Yoon, J.H. Yang, H.H. Kwon, S. Min, D.H. Suh, Atrophic acne scar: a process from altered metabolism of elastic fibres and collagen fibres based on transforming growth factor-β1 signalling, British Journal of Dermatology, Volume 181, Issue 6, 1 December 2019, Pages 1226-1237].

A key atopic dermatitis pathomechanism is Th2 lymphocyte activation in inflammatory process. The release of numerous cytokines and growth factors, such as IL-1, IL-4, IL-6, IL-13, TGF-β, and TNF, by immune cells can promote the destruction of extracellular matrix by activation of MMP-8 and MMP-9. In this connection the patients with atopic dermatitis are characterized by elevated serum MMP-8 and MMP-9 concentrations [Bhattacharjee, O.; Ayyangar, U.; Kurbet, A.S.; Ashok, D.; Raghavan, S. Unraveling the ECM-Immune Cell Crosstalk in Skin Diseases. Front. Cell Devel. Biol. 2019] and damaged type I collagen. Furthermore, increased macrophage infiltration in chronic phase of the disease can also disturb the extracellular matrix homeostasis due to increased MMP activity. The disturbance of extracellular matrix structure due to lower type I collagen synthesis results in increased permeability, impaired cohesion and integrity of epidermis, higher transepidermal loss of moisture, dry skin, and appearance of skin cracks and skin ruptures [Krzysztof Szalus, Magdalena Trzeciak, The Role of Collagens in Atopic Dermatitis, Int. J. Mol. Sci. 2024, 25(14), 7647].

In the oral cavity a bacterial biofilm serves as a key factor of periodontal diseases. Gram-positive and Gram-negative bacteria have numerous structural or released components, which can induce direct destruction of periodontal tissues or stimulate the cells to activate a wide range of inflammatory responses [Madianos, P.N., Bobetsis, Y.A. and Kinane, D.F. (2005), Generation of inflammatory stimuli: how bacteria set up inflammatory responses in the gingiva. Journal of Clinical Periodontology, 32: 57-71]. Since nonspecific (innate) component of immune response is not directed specifically to a certain antigen, the immune response is harmful not only to microbial components but also to the tissues supporting the teeth. Degradation of said tissues lead to clinically apparent symptoms: gingival hemorrhage, increased depth of periodontal pouch, tooth loosening, and finally to edema. It is believed that approximately 80% cases of periodontal damage emerge from the secondary damage caused by immune response [N 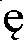 dzi-Góra, M., Kowalski, J. & Górska, R. The Immune Response in Periodontal Tissues. Arch. Immunol. Ther. Exp. 65, 421-429 (2017). https://doi.org/10.1007/s00005-017-0472-8].

In such diseases as periodontitis there are several mechanisms of destruction and slow synthesis of collagen fibers. Normally type I collagen synthesis is regulated by autophagia process; however, it was shown that periodontopathogenic bacteria (*Porphyromonas gingivalis*) are able to inhibit autophagia. Because of this damaged or incorrectly folded collagen is accumulated in tissues instead of eliminating; this results in disturbed synthesis of extracellular matrix, slow synthesis of normal collagen molecules, and prevents the osteoblastic cell differentiation. All of these things slow the tissue regeneration and wound healing processes in the oral cavity [Nakamura, T., Yamashita, M., Ikegami, K. et al. Autophagy facilitates type I collagen synthesis in periodontal ligament cells. Sci Rep 11, 1291 (2021)]. Margin epithelium forms a unique membrane between the root surface and a gum, and its primary function is to protect the underlying tissues from continuous exposure to the microorganisms in the oral cavity and to the side products thereof. If this protective system is overwhelmed by the bacterial (for example, *P. gingivalis*) virulence factors and persistent inflammation (which is manifested clinically as a gingival hemorrhage and altered contours and color of soft tissues, periodontitis), the connective epithelium migrates upward over the root surface and activates the collagen destruction that finally leads to formation of periodontal pouches. In disease MMP-8 is the main collagenase that actively promotes the destruction of periodontal tissues [Könönen, E.; Gursoy, M.; Gursoy, U.K. Periodontitis: A Multifaceted Disease of Tooth-Supporting Tissues. J. Clin. Med. 2019, 8, 1135].

TRPV channels expressing in skin keratinocytes, sensory neurons, melanocytes and immune cells localized in epidermis, derma and hypoderm are no less important. Said channels are involved in physiological processes including transmittance of sensations and thus maintain the skin homeostasis. Both excessive and inadequate activity of TRPV channels may induce abnormal conditions, such as chronic pain and pruritus, dermatitis, vitiligo, alopecia, skin carcinogenesis, and the skin barrier disturbance [Okada, Yuka, et al. "Roles of epithelial and mesenchymal TRP channels in mediating inflammatory fibrosis." Frontiers in Immunology 12 (2022): 731674.]. Exposure to proinflammatory cytokines results in considerable activation of TRPV1 channel [Planells-Cases, R., Garcia-Sanz, N., Morenilla-Palao, C. et al. Functional aspects and mechanisms of TRPV1 involvement in neurogenic inflammation that leads to thermal hyperalgesia. Pflugers Arch - Eur J Physiol 451, 151-159 (2005)]. High activation of TRPV1 is one of potential mechanisms reducing the synthesis and accumulation of type I collagen [Yu Cao, Yongju Wen, Zongyuan Zhou, Ruiying Xi, Wen Shuai, Jichao Zhang, Apichart Suksamrarn, Guolin Zhang, Xiao-xia Lu, Fei Wang. Discovery of novel capsaicin analogs as TRPV1 inhibitors for the treatment of idiopathic pulmonary fibrosis, European Journal of Medicinal Chemistry, 2025, 284, 117229], and presents an innovative mechanism. In addition, TRPV1 mediates the tissue inflammation and destruction in oral cavity diseases. TRPV1 was found to be important for pain signal transmittance in pulp, it induces the inflammation and is involved in bone tissue resorption in periodontitis [Evangeliou, Evangelos, et al. "Impact of TRP channels in Oral pathology and therapeutic targeting options: a narrative review." Current Topics in Medicinal Chemistry 23.16 (2023): 1559-1573.].

Matrix metalloproteinases (MMP) play a key role in destruction of basal membranes and type I collagen. It has been established that MMP-9 is activated by bacterial infection, and in this situation accelerated migration and invasion of the infected cells is observed. As was shown previously, LPS is a key virulence factor and the stimulatory factor for MMP-9 cell production under the bacterial invasion [Inaba, H., Sugita, H., Kuboniwa, M., Iwai, S., Hamada, M., Noda, T., Morisaki, I., Lamont, R.J. and Amano, A. (2014), Promotion of oral cancer invasion by P. gingivalis. Cell Microbiol, 16: 131-145]. Collagenases MMP-1, MMP-8, MMP-13, and MMP-18 are able to break down interstitial type I, II, III collagens giving rise to hydrolyzed collagen or gelatin [Luchian, I.; Goriuc, A.; Sandu, D.; Covasa, M. The Role of Matrix Metalloproteinases (MMP-8, MMP-9, MMP-13) in Periodontal and Peri-Implant Pathological Processes. Int. J. Mol. Sci. 2022, 23, 1806]. Elevated MMP level is associated with propagation of periodontal damage, progression of attachment losses and deepening of the periodontal pouches [Luchian, I.; Goriuc, A.; Sandu, D.; Covasa, M. The Role of Matrix Metalloproteinases (MMP-8, MMP-9, MMP-13) in Periodontal and Peri-Implant Pathological Processes. Int. J. Mol. Sci. 2022, 23, 1806]. In the patients with periodontitis salivary MMP-8 levels and crevicular MMP-9 levels are significantly higher compared to the patients with gingivitis and healthy adults. Salivary MMP-8 level and crevicular MMP-9 levels can serve as the biomarkers of the periodontal disease and be useful for early diagnosis of periodontitis or gingivitis [Rai, Balwant, et al. "Biomarkers of periodontitis in oral fluids." Journal of oral science 50.1 (2008): 53-56.]. Therefore, high MMP level in skin resulted from uncontrolled microbial invasion results in poor skin quality and decrease in skin firmness because of decrease in the number of collagen fibers.

Since sustained skin and mucosa health is considerably dependent on type I collagen, there is an obvious need in maintenance of type I collagen level for the prevention of skin and oral diseases. Though peptide molecules are able to stimulate type I collagen synthesis in derma, said molecules are not stable and show low penetration into the skin integuments and derma because are broken down by the skin endoproteases [Fosca Errante, Marta Menicatti, Marco Pallecchi, Lisa Giovannelli, Anna Maria Papini, Paolo Rovero, Gianluca Bartolucci, Susceptibility of cosmeceutical peptides to proteases activity: Development of dermal stability test by LC-MS/MS analysis, Journal of Pharmaceutical and Biomedical Analysis, Volume 194,2021, ISSN 0731-7085]. Widely used antiseptic agents, such as chlorohexidine bigluconate, exhibit only antibacterial activity and are associated with development of antimicrobial resistance of skin and oral microbiota [Singkham-In, U.; Phuengmaung, P.; Makjaroen, J.; Saisorn, W.; Bhunyakarnjanarat, T.; Chatsuwan, T.; Chirathaworn, C.; Chancharoenthana, W.; Leelahavanichkul, A. Chlorhexidine Promotes Psl Expression in Pseudomonas aeruginosa That Enhances Cell Aggregation with Preserved Pathogenicity Demonstrates an Adaptation against Antiseptic. Int. J. Mol. Sci. 2022, 23, 8308]. In view of the above reasons, chlorohexidine and similar antibiotics are not recommended for the regular use in perfumery and cosmetic products because of the risk of developing resistance and dysbiosis of skin and mucosa in the event of microbial invasion of the soft tissues. Anti-inflammatory glucocorticosteroids were also considered as the potential components, however, said agents are not able to restore type I collagen synthesis in derma and mucosa and only contribute to development of side effects, such as skin atrophy, erosions, slow wound healing, purpura, liability to bruises and acne [Oray, M., Abu Samra, K., Ebrahimiadib, N., Meese, H., & Foster, C. S. (2016). Long-term side effects of glucocorticoids. Expert opinion on drug safety, 15(4), 457-465]. The use of retinol and retinol derivatives promotes type I collagen synthesis though continuous administration of said agents is associated with irritant contact dermatitis, pruritus, burning and skin hypersensitivity, erythema, skin peeling, and cheilosis [Albanova V.I. The role of local retinoids in eliminating signs of skin aging // Vestnik dermatologii i venerologii. - 2021. - Vol. 97. - N. 4. - P. 60-70]. The use of retinoids in beauty and pharmaceutical products is limited by irritative properties of these agents [Albanova V.I. The role of local retinoids in eliminating signs of skin aging // Vestnik dermatologii i venerologii. - 2021. - Vol. 97. - N. 4. - P. 60-70. doi: 10.25208/vdv1220].

Thus, the problem of tissue destruction because of damage and the absence of safe, efficacious, and bioavailable options of supporting type I collagen synthesis based on the novel mechanisms is urgent and yet unsolved.

Paeoniflorin, a water-soluble monoterpene glycoside isolated from the roots of *Paeonia lactiflora,* is a promising component. The studies showed that paeoniflorin exhibits a wide range of *in vitro* and *in vivo* pharmacological effects including anti-inflammatory, antioxidant, antithrombotic, anticonvulsive, analgesic, cardioprotective, neuroprotective, hepatoprotective and antidepressant-like effects [Zhou, Y. X., Gong, X. H., Zhang, H., & Peng, C. (2020). A review on the pharmacokinetics of paeoniflorin and its anti-inflammatory and immunomodulatory effects. Biomedicine & Pharmacotherapy, 130. 110505.]. Due to anti-inflammatory and immunoregulatory effects paeoniflorin and the other peony bioactive compounds are considered as the active ingredients for treating rheumatoid arthritis, psoriasis, and dermatitis, which is capable to regulate the signaling pathways (GPCR, MAPK/NF-xB pathway, PI3K /Akt /mTOR pathway, JAK2/STAT3 pathway, TGFβ /Smads pathway [Zhang, L., & Wei, W. (2020). Anti-inflammatory and immunoregulatory effects of paeoniflorin and total glucosides of paeony. Pharmacology & therapeutics, 207, 107452].

It is important that paeoniflorin was proposed by the authors in the context of elimination of consequences of type I collagen degradation in the skin and oral soft tissues, which is based on the effect on targets associated with the tissue inflammation and destruction. The authors found in the experiments that paeoniflorin increases type I collagen production in the tissues of skin and oral mucosa thus opening the prospects for preventing, treating and alleviating of such diseases and abnormal conditions as atopic dermatitis, acne, psoriasis, periodontitis, periodontosis, etc. In addition, the experiments by the authors supported the previously studied paeoniflorin effect on the known targets, in particular, on TRVP1, MMP8, MMP9, TNFα and some interleukins [Zhou YX, Gong XH, Zhang H, Peng C. A review on the pharmacokinetics of paeoniflorin and its anti-inflammatory and immunomodulatory effects. Biomed Pharmacother. 2020 Oct;130:110505] and revealed a new interrelation between the type I collagen synthesis and modulation by MMP-8, MMP-9 and TRPV1 channels. These experiments not only open up new prospects for the use of peoniflorin, but also demonstrate an entirely new field of use, as an active ingredient maintaining the level of type I collagen to prevent the tissue destruction resulting from bacterial invasion and an acute inflammatory response, which, as mentioned above, is an acute problem for both skin tissues and oral mucosa. The affect is achieved mainly through regulation of expression of genes encoding the enzymes involved in type I collagen synthesis, decrease in the inflammatory factor levels and down-regulation of TRPV-1 channels, which are involved in the response to the virulent agent invasion. The response and overly active immune response lead to the tissue destruction and subsequent inflammation contributing to development of hyperreactivity, i.e. hypersensitivity to pain stimuli (hyperalgesia) or painful sensation in response to painless stimuli (allodynia). Desensitization of TRPV1 as paeoniflorin nociceptor provides the relief from hyperreactivity, unpleasant pain symptoms, and reduces the tissue destruction under the bacterial invasion.

In contrast to the above components, the Authors of the present invention investigated and for the first time established paeoniflorin efficacy and capability to restore the structure of soft tissues, mucosa, and epithelial integuments by promoting the synthesis and restoration of the amount of type I collagen to the basal level. The component is active over pH range from 5.0 to 8.0. which is consistent with the most areas of oral, nasal, and ocular (conjunctival) mucosa, and epidermis of skin, scalp, and exocrine glands. This phytocomponent does not disturb the cell structures throughout the exposure, promotes the collagen I production, and helps to maintain and repair the damaged skin and mucosal integuments. Furthermore, the component works upon inherent epigenetic regulatory processes of TRPV1 channels and restores the normal sensation threshold in cells.

The use of the component helps to provide the treatment and/or prevention of various diseases, which are mediated by destruction or low synthesis of type I collagen fibers due to bacteria invasion in various tissues and subsequent inflammation, to ameliorate the condition of skin and oral, nasal, ocular mucosa. Furthermore, paeoniflorin is a 100% natural, biodegradable component, which is free of ingredients of animal origin.

### SUMMARY OF THE INVENTION

In the first aspect the invention is related to paeoniflorin to take care of body and head skin, oral, nasal, ocular, and secretory mucosa, in particular, to alleviate the irritation of soft tissues, mucosa and epithelial integuments caused by type I collagen destruction when invaded by the virulent agents. The invention is related to paeoniflorin of plant origin, which can be found in roots, rhizomes, leaves, herbs and/or flowers, wherein the component level expressed as the active ingredient is 0.001 wt% to 0.08 wt%.

The invention is distinguished by that paeoniflorin (Paeoniflorin or Peoniflorin) is a substance under registration number CAS 23180-57-6. Paeoniflorin may be available as paeoniflorin of plant origin, such as *Peoniflorin, Paeoniflorin, White Peony Extract, Paeonia lactiflora root extract,* standardized by paeoniflorin content, or other commercially available raw materials articles.

Paeoniflorin of invention may be available as various paeoniflorin structural isomers, the mixture thereof, or as a plant extract comprising all paeoniflorin forms.

The composition of the invention can comprise, % by weight:

| | |
|---|---|
| Paeoniflorin *Peoniflorin* | 0.001-0.08%. |

In the second aspect the invention is related to the use of paeoniflorin to alleviate the irritation of soft tissues, mucosa and epithelial integuments resulted from destruction of type I collagen caused by the virulent agent invasion.

In the third aspect the invention is related to the use of a composition of the invention to reduce the active tissue destruction resulted from type I collagen destruction under bacterial invasion of skin tissues and oral mucosa.

In the fourth aspect the invention is related to the use of a composition of the invention to reduce the hypersensitivity and pain sensitivity and to prevent the diseases affecting mucosa and epidermis, for alleviation of disease-associated conditions through stimulation of type I collagen production in tissues and reduction in number of TRPV1 channels.

In the fifth aspect the invention is related to the face and body care products for the skin prone to atopic dermatitis, eczema, contact dermatitis, in particular, to lotions, creams, sera, cleansing gels, masks, spray-mists, gels, oleogels, balms, butters, hydrophilic oils comprising paeoniflorin of the invention in the amount of 0.001 wt% to 0.08 wt%.

In the sixth aspect the invention is related to the oral care products, in particular, to the toothpastes, tooth gels, teething gels, oral rinsing gels, moisturizing gels, oral sera, reabsorbable formulations, solid powders comprising *paeoniflorin* of the invention in the amount of 0.001 wt% to 0.08 wt%.

In the eighth aspect the invention is related to the scalp care products, in particular, to the scalp and hair shampoos, antidandruff shampoos and shampoos for seborrheic dermatitis and scalp xerosis, lotions, creams, sera, gels, cleansing products comprising *paeoniflorin* of the invention in the amount of 0.001 wt% to 0.08 wt%.

In the ninth aspect the invention is related to the face and body cleansing products such as washing gel, exfoliating gel, cleansing foam-mousse, micellar water, cleansing liquid soap for body, hand and face, solid soap comprising *paeoniflorin* of the invention in the amount of 0.001 wt% to 0.08 wt%.

### DETAILED DESCRIPTION OF THE INVENTION.

In the first aspect the invention is related to paeoniflorin to take care of body and head skin, oral, nasal, ocular, and secretory mucosa, in particular, to alleviate the irritation of soft tissues, mucosa and epithelial integuments caused by the invasion of the virulent agents. The invention is related to paeoniflorin of plant origin, which can be found in roots, rhizomes, leaves, herbs and/or flowers, wherein the component level expressed as the active ingredient is 0.001 wt% to 0.08 wt%.

The invention is distinguished by that paeoniflorin (Paeoniflorin or Peoniflorin) is a substance under registration number CAS 23180-57-6. Peoniflorin (INCI: Peoniflorin 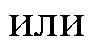 Paeonia Lactiflora Root Extract) is a commercially available ingredient.

Paeoniflorin of the invention may be available as paeoniflorin of plant origin, such as *Peoniflorin, Paeoniflorin, White Peony Extract, Paeonia lactiflora root extract,* standardized by paeoniflorin content, or other commercially available raw materials articles.

Paeoniflorin of invention may be available as various paeoniflorin structural isomers, the mixture thereof, or as a plant extract comprising all paeoniflorin forms. Said molecules are well-known for those skilled in the art and are commercially available from various suppliers.

The composition of the invention can comprise, % by weight:

| | |
|---|---|
| Paeoniflorin *Peoniflorin* | 0.001-0.08%. |

In the product of the invention the acceptable excipients can be selected from the following component categories: anionic surfactants, amphoteric surfactants, non-ionic surfactants, dispersing media and solvents, pH adjusting agents, abrasive components, bleaching components, remineralizing components, enzymes, thickeners, sweeteners and sugar substitutes, complexing agents (chelating agents), emulsifiers forming direct and inverse emulsions, emollients, vegetable oils, mineral salts, silicones, synthetic polymers, film-forming components, plant extracts, preservatives, flavoring agents, essential oils, pigments.

### EXPERIMENTS

The examples in this specification shall not be considered as limiting the present invention, and are included merely for illustrative purpose and to support the achievement of the expected technical results. These examples are among many experimental findings obtained by the Authors of invention, which prove the efficacy of the products within the scope of invention.

### Example 1. In vitro evaluation of paeoniflorin cytotoxicity and safety profile in human keratinocytes.

Human keratinocytes were seeded into 75cm² flasks (Corning, USA), cultured and propagated in incubator at 37°C in the presence of 5% CO₂ with the use of a culture-specific medium. Upon the fusion the keratinocytes were transferred to 96-well plates (Corning, USA) to evaluate the safe concentrations. The cell viability was determined by colorimetry with the use of MTT dye (Sigma Chemical, USA). Test samples prepared in culture medium were transferred in 96-well plate and serial dilutions from 100 to 0.03 mg/mL with dilution coefficient 3,16⁹ were made. Keratinocyte culture was incubated for 48 hours followed by addition of MTT at 0.5 mg/mL (100 µL/well), and incubation was continued for more 4 hours. The well was emptied, and 100 µL of isopropanol was added to dissolve the formed formazan crystals. The measurements of optical absorption were made for each well at 570 nm using Multiskan GO monochromator (Thermo Scientific, Finland).

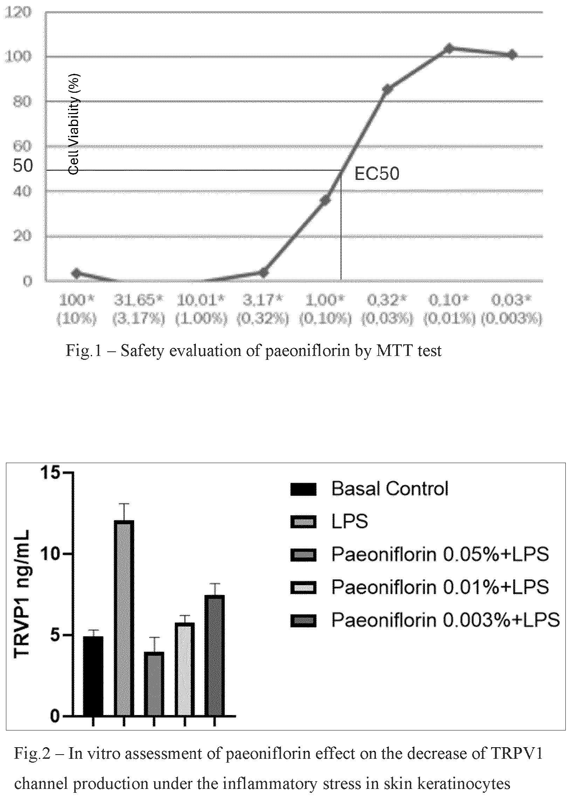

Considering said MTT test cytotoxicity data, it can be assumed that there is no relevant toxicity in any case up to 0.08 wt.% paeoniflorin, and therefore the following exemplarily safe concentrations covering said range have been used in the subsequent examples: 0.003 wt.%, 0.01 wt.% and 0.05 wt.% paeoniflorin (see Fig.1).

### Example 2. In vitro assessment of paeoniflorin effect on the number of TRPV1 sensitivity channels in human keratinocytes.

The human keratinocytes were treated with paeoniflorin at three safe concentrations (0.05%, 0.01%, 0.003%) in the presence of LPS (toxins, which are localized on the external membrane of a bacterial cell and can induce the inflammation) 50 µg/mL for four days. TRPV-1 was measured in the supernatant and the cell lysate by ELISA enzyme immunoassay. The LPS 50 µg/mL exposure to the cell culture contributed to the increase of TRPV-1 synthesis by 144.45% (P<0.001) suggesting acute inflammatory response and increase in the skin sensitivity.

In keratinocytes the nociceptors such as TRPV1 are found. Nociceptors are the sensory fibers responding to the stimuli, which are potentially harmful for the organism. This specialized protein plays a key role in our sensory perception especially in response to temperature, pain and various environmental stimuli. TRPV1 mediates pro-inflammatory effects in the oral cavity, which lead to periodontium damage. In addition, TRVP-1 activation is also a signal of skin sensitization, and various methods of down-regulation of TRVP-1 expression, such as neuropeptide depletion or calcium inflow for the calcium-dependent proteins are considered as therapeutic approaches to decrease the pain and sensitivity [Bagood, M. D., & Isseroff, R. R. (2021). TRPV1: Role in Skin and Skin Diseases and Potential Target for Improving Wound Healing. International Journal of Molecular Sciences, 22(11), 6135. https://doi.org/10.3390/ijms22116135].

Therapeutic effect was demonstrated for all three paeoniflorin concentrations 0.05%, 0.01%, and 0.003%, which decreased the overproduction of TRPV1 channels in the skin cells by 113.47%, 88.40%, and 64.41% respectively (P<0.001) (see Fig. 2). These results highlighted the role of peoniflorin as targeted substance in action to TRPV1 channeks related to itch, increased sensitivity, and pain sensation.

Paeoniflorin 0.05% provides 100% inhibition of TRPV1 overproduction (compared to the dirrefence between basal control and LPS-induced control) under LPS activation suggesting that paeoniflorin completely suppresses the inflammatory process and can have a therapeutic effect in the event of inflammation.

### Example 3. In vitro investigation of the effect of non-toxic paeoniflorin concentrations on type I collagen synthesis in dermal fibroblasts

The fibroblast cultures were seeded and kept for 48 hours in special culture medium and were propagated in incubator at 37°C in the presence of 5% CO₂. On Day3 the culture supernatant was collected for the assay of type I collagen, then the fibroblast culture was treated with paeoniflorin at 0.50, 0.10, and 0.03 mg/mL and with green tea extract at 0.0079, 0.005, and 0.0008 mg/mL and exposed to LPS 50 µg/mL for additional 48 hours. On Day 5 supernatant and the cell lysate were collected for the assay of type I collagen.

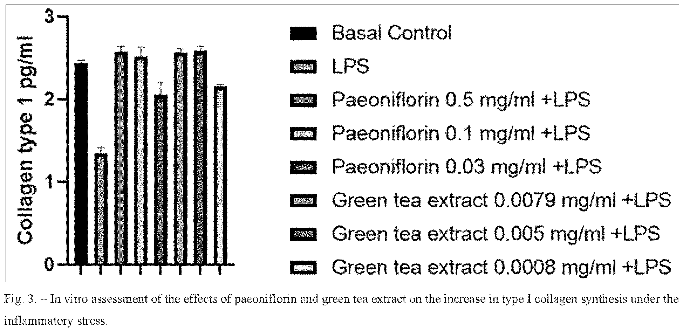

The Figures 3 show the results of comparative efficacy analysis of paeoniflorin and green tea extract, which measured type I collagen production in the cultured fibroblasts. On Day 3 no significant between-group difference was observed (P>0.05), therefore the comparison was made on Day 5. As expected, LPS exposure resulted in the decrease of type I collagen production in the cell culture by 35.87% (P<0.001) compared to the basal control group. As is evident from Fig. 3, paeoniflorin at 0.50. 0.10 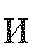 0.03 mg/mL showed the increase in type I collagen production by 92.16% (P<0.001), 87.50% (P<0.001), and 52.82% (P<0.001), respectively compared to LPS treatment group. Similarly, the green tea extract at 0.0079, 0.005 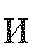 0.0008 mg/mL demonstrated the increase in type I collagen production by 90.72% (P < 0.001), 92.58% (P < 0.001) and 60.21% (P < 0.001), respectively, compared to LPS treatment group (see Fig.8).

### Example 4. In vitro investigation of the effect of non-toxic paeoniflorin concentrations on type I collagen synthesis in gingival fibroblasts.

The fibroblast cultures were seeded and kept for 48 hours in special culture medium and were propagated in incubator at 37°C in the presence of 5% CO₂. On Day 3 the culture supernatant was collected for the assay of type I collagen, then the fibroblast culture was treated with paeoniflorin at 0.50, 0.10, and 0.03 mg/mL and with green tea extract at 0.0079, 0.005, and 0.0008 mg/mL and exposed to LPS 50 µg/mL for additional 48 hours. On Day 5 supernatant and the cell lysate were collected for the assay of type I collagen.

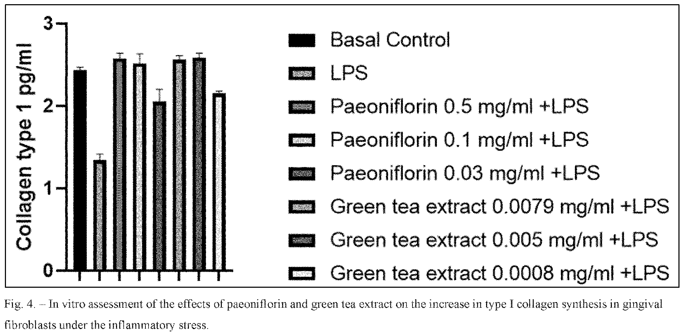

Figure 4 shows the results of comparative efficacy analysis of paeoniflorin and green tea extract, which measured type I collagen production in the cultured gingival fibroblasts. On Day 3 no significant between-group difference was observed (P>0.05), therefore the comparison was made on Day 5. As expected, LPS exposure resulted in the decrease of type I collagen production in the cell culture by 44.78% (P<0.001) compared to the basal control group.

As is evident from Fig. 4, paeoniflorin at 0.50. 0.10 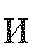 0.03 mg/mL showed the increase in type I collagen production by 92.16% (P<0.001), 87.50% (P<0.001), and 52.82% (P<0.001), respectively, compared to LPS treatment group. On the other hand, we can express the obtained results as percentage of recovery after the LPS exposure. This means that paeoniflorin at 0.50. 0.10 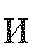 0.03 mg/mL showed the recovery of type I collagen by 113.64%, 107.89%, and 65.13%, respectively. Similarly, the green tea extract at 0.0079, 0.005 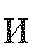 0.0008 mg/mL demonstrated the increase in type I collagen production by 90.72% (P < 0.001), 92.58% (P < 0.001) and 60.21% (P < 0.001), respectively, compared to LPS treatment group (see Fig. 10) with the recovery of type I collagen by 111.86%, 114.15%, and 74.24% at three tested concentrations.

### Example 5. In vitro assessment of paeoniflorin effect on the synthesis of TNF-α pro-inflammatory cytokine in human keratinocytes.

Human keratinocytes were treated with paeoniflorin at three safe concentrations (0.05%, 0.01%, 0.003%) in the presence of bacterial pro-inflammatory component (LPS 50 µg/mL) for 4 days. The concentration of TNF-α pro-inflammatory cytokine was measured in the collected cell supernatant by ELISA enzyme immunoassay. Exposure to LPS (50 µg/mL) contributed to the increase in TNF-α pro-inflammatory cytokine synthesis by 25.64% (P<0.01) in skin keratinocytes suggesting the onset of acute inflammatory response and immune response.

In oral cavity the gingival epithelium keratinocytes form the barrier against bacterial infection and invasion. The keratinocytes are able to produce various pro-inflammatory cytokines and chemokines including interleukin (IL)-1, IL-6, IL-8, and the tumor necrosis factor (TNF)-α [https://microbiol.crie.ru/jour/issue/download/3/2]. The synthesis of the above cytokines can be induced by infections caused by pathogenic bacteria, such as *Porphyromonas gingivalis* (*P. gingivalis*) and *Aggregatibacter actinomycetemcomitans* (*A. actinomycetemcomitans*). In skin TNF-α induces inflammation and, more importantly, reduces transepithelial electrical resistance thus increasing the skin permeation and promoting serious problems with skin moisturizing [Choi, D.H., Hwang, H.S. Anti-inflammation activity of brazilin in TNF-α induced human psoriasis dermatitis skin model. Appl Biol Chem 62, 46 (2019). https://doi.org/10.1186/s13765-019-0455-z].

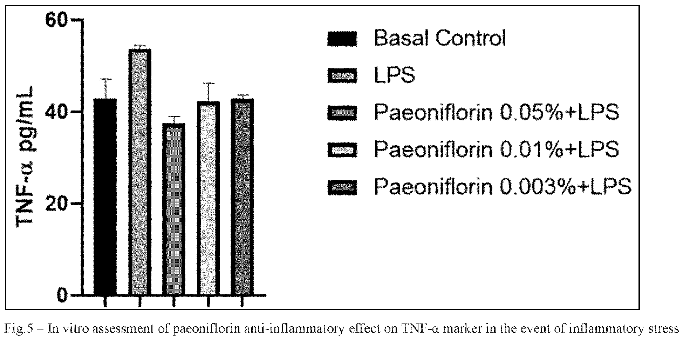

Paeoniflorin at concenrations of 0.05%, 0.01%, 0.003% decreased the inflammatory stress decreasing TNF-α overproduction by 148.82% (P<0.001), 105.49% (P<0.01), and 100.00% (P<0.01) respectively, thus demonstrating marked anti-inflammatory response (see Fig. 5). Paeoniflorin 0.05% completely eliminated the LPS-induced inflammatory stress, i.e. decreased TNF-α overproduction by 148.82% (p<0.001).

### Example 6. In vitro assessment of paeoniflorin effect on IL-6 synthesis in human keratinocytes.

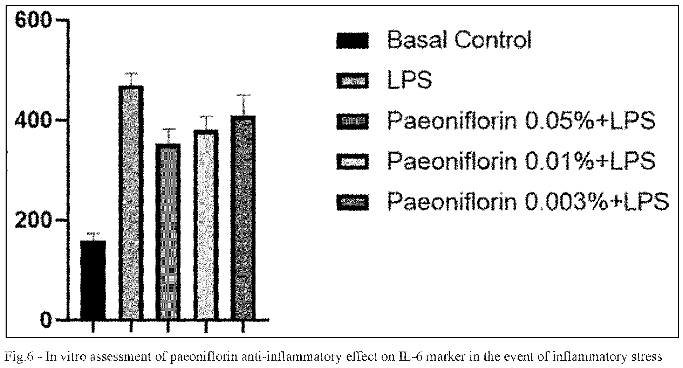

Human keratinocytes were treated with paeoniflorin at three safe concentrations (0.05%, 0.01%, 0.003%) in the presence of pro-inflammatory component isolated from bacteria (LPS 50 µg/mL) for 4 days. IL-6 is involved in emergence and propagation of chronic inflammation [Barnes, T. C., Anderson, M. E., & Moots, R. J. (2011). The many faces of interleukin-6: the role of IL-6 in inflammation, vasculopathy, and fibrosis in systemic sclerosis. International journal of rheumatology, 2011(1), 721608.]. The IL-6 level was measured in the collected cell supernatant by ELISA enzyme immunoassay. Exposure to LPS (50 µg/mL) contributed to the increase in IL-6 synthesis by 194.20% (P<0.001) in skin keratinocytes. In the skin affected by psoriasis markedly elevated IL-6 level and the most intensive expression is observed [Goodman, W. A., Levine, A. D., Massari, J. V., Sugiyama, H., McCormick, T. S., & Cooper, K. D. (2009). IL-6 signaling in psoriasis prevents immune suppression by regulatory T cells. The Journal of Immunology, 183(5), 3170-3176.]. In addition, proinflammatory cytokines including IL-6 are involved in periodontitis pathogenesis and serve as inflammatory markers in the oral cavity [Reis, C., Da Costa, A. V., Guimarães, J. T., Tuna, D., Braga, A. C., Pacheco, J. J., ... & Cardoso, E. M. (2014). Clinical improvement following therapy for periodontitis: Association with a decrease in IL-1 and IL-6. Experimental and therapeutic medicine, 8(1), 323-327.] Paeoniflorin in concentrations of 0.05% and 0.01%, demonstrated protective and anti-inflammatory effects with IL-6 level decrease by 37.78% and 28.70% in keratinocytes (see Fig. 6).

### Example 7. In vitro assessment of paeoniflorin effect on IL-13 synthesis in human keratinocytes

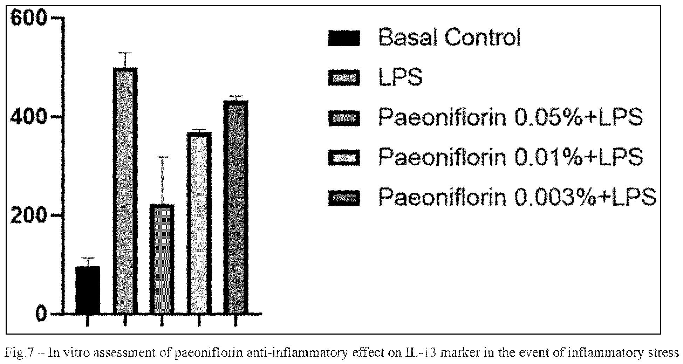

Human keratinocytes were treated with paeoniflorin at three safe concentrations (0.05%, 0.01%, 0.003%) in the presence of pro-inflammatory component isolated from bacteria (LPS 50 µg/mL) for 4 days. Pro-inflammatory cytokine IL-13 was chosen as a marker, because the elevated IL-13 level is found in skin lesions associated with atopic dermatitis [Oh, M. H., Oh, S. Y., Yu, J., Myers, A. C., Leonard, W. J., Liu, Y. J., ... & Zheng, T. (2011). IL-13 induces skin fibrosis in atopic dermatitis by thymic stromal lymphopoietin. The Journal of Immunology, 186(12), 7232-7242]. The IL-13 level was measured in the collected cell supernatant by ELISA enzyme immunoassay. Exposure to LPS (50 µg/mL) contributed to the increase in IL-13 synthesis by 409.75% (P<0.001) in skin keratinocytes this simulating a chronic immune response and inflammatory response in the skin affected by atopic dermatitis. IL-13 is a key cytokine of type 2 immunity, which induces peripheral inflammation. IL-13 is characterized by local overexpression and exhibits a considerable influence on skin biology including inflammatory cell recruiting, alteration of skin microbiome and impairment of the epidermal barrier functioning [Bieber, T. (2020). Interleukin-13: targeting an underestimated cytokine in atopic dermatitis. Allergy, 75(1), 54-62.]. Paeoniflorin in concentraions of 0.05%, 0.01% demonstrated protective and anti-inflammatory effects with IL-13 level decrease by 68.83% and 32.61% in keratinocytes (see Fig. 7).

The results of all investigations clearly show the pronounced triple anti-inflammatory effect of low paeoniflorin concentrations in the event of inflammatory stress associated with immune response in the skin affected by various dermatological conditions and diseases. The integrated anti-inflammatory effect provides the multifaceted protection of the skin cells from the inflammatory stress caused by bacteria and LPS, the immune cells and pro-inflammatory cytokines.

### Example 8. Assessment of paeoniflorin stability by HPLC/UV assay when used as a rinse ingredient

Storage stability of paeoniflorin as a mouthwash ingredient was tested (Table 1).

### Procedure

*Mobile phase A:* 160 mL of acetonitrile was transferred to IL volumetric flask and made up to the volume with water, then 0.5mL of trifluoracetic acid (conc.) was added.

*Mobile phase B:* 950 mL of acetonitrile was transferred to IL volumetric flask and made up to the volume with water, then 0.5mL of trifluoracetic acid (conc.) was added.

*Reference solution:* 5 mg of 50% paeoniflorin was transferred into 10 mL volumetric flask, dissolved in 5 mL of methanol and made up to the volume with the same solvent, then mixed (0.5 mg/mL).

*Test solution:* No special sample preparation.

Chromatographic conditions is shown in Table 1.

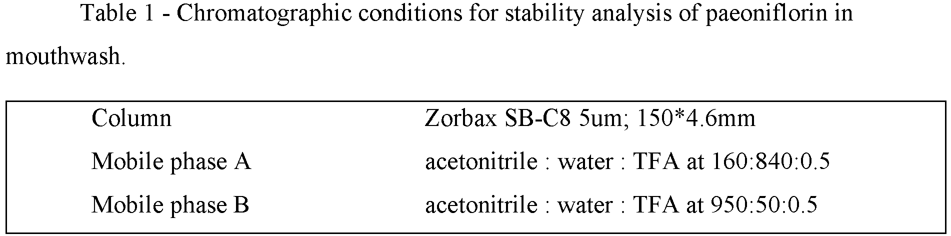

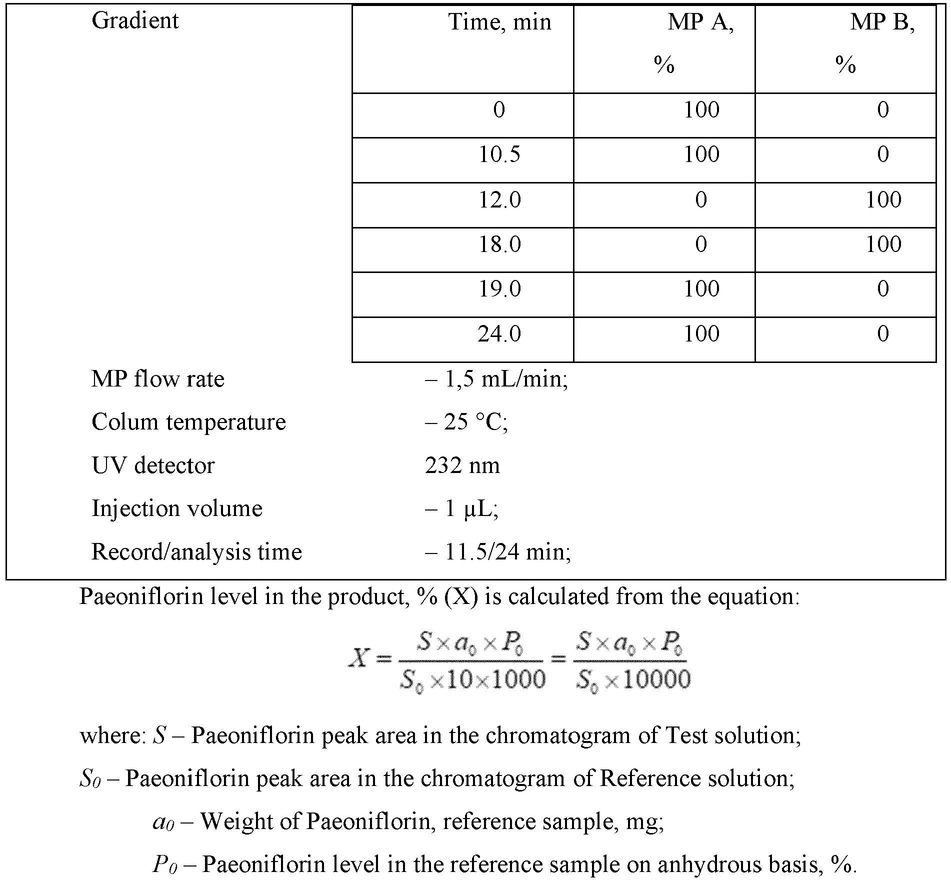

The results of paeoniflorin assay calculations are shown in Table 2.

**Table 2 - Results of paeoniflorin assay under different conditions.**

| 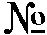 | Storage conditions | Compound | tR | Peak Area | Relative content |
|---|---|---|---|---|---|
| Baseline paeoniflorin assay | | | | | 100% |
| 1 | Room 14 days | Paeoniflorin-1 (isomer) | 4.15 | 101943.1 | 100% |
| | | Paeoniflorin-2 (isomer) | 4.71 | 254206.4 | 100% |
| 2 | Thermo 14 days | Paeoniflorin-1 (isomer) | 4.15 | 95042.54 | 93% |
| | | Paeoniflorin-2 (isomer) | 4.71 | 221843.4 | 87% |
| 3 | Thermo 40 days | Paeoniflorin-1 (isomer) | 4.14 | 94923.67 | 93% |
| | | Paeoniflorin-2 (isomer) | 4.7 | 220621.7 | 87% |

The results show that stability of paeoniflorin as a rinse ingredient was maintained during the storage for 40 days under accelerated (thermo) conditions, which were consistent with the product storage for 1 year; the decrease of the substance assay was up to 10%, which is insignificant and allows to use paeoniflorin in the long-life products.

When the green tea extract was used the instability and degradation was observed both due to light and oxygen instability of the green tea [Jahanfar, S., Ghavami, M., Khosravi-Darani, K., & Jahadi, M. (2021). Liposomal green tea extract: optimization and physicochemical characterization. Journal of Applied Biotechnology Reports, 8(1).], and due to instability of epigallocatechin gallate (principal active ingredient of the extract), which led to production of degradation products and pro-oxidant molecules exhibiting various side effects, rather than just resulted in lower bioavailability [Krupkova, O., Ferguson, S. J., & Wuertz-Kozak, K. (2016). Stability of (-)-epigallocatechin gallate and its activity in liquid formulations and delivery systems. The Journal of nutritional biochemistry, 37, 1-12., Granja, A., Frias, I., Neves, A. R., Pinheiro, M., & Reis, S. (2017). Therapeutic potential of epigallocatechin gallate nanodelivery systems. BioMed Research International, 2017(1), 5813793., Mehmood, S., Maqsood, M., Mahtab, N., Khan, M. I., Sahar, A., Zaib, S., & Gul, S. (2022). Epigallocatechin gallate: Phytochemistry, bioavailability, utilization challenges, and strategies. Journal of Food Biochemistry, 46(8), e14189.].

## Claims

1. A composition formulated for application on and/or to a subject's connective soft tissue, wherein said composition comprises paeoniflorin.

2. The composition of claim 1, wherein said formulation for application is a formulation for topical application.

3. The composition of claim 1 or claim 2, wherein said composition comprises 0.001% to 0.080% paeoniflorin by weight of said composition, wherein preferably said composition comprises 0.001% to 0.050% paeoniflorin by weight of said composition, including 0.003%, 0.010% and 0.050% by weight of said composition.

4. The composition of any of the preceding claims, wherein said connective soft tissue comprises type I collagen and/or wherein said connective soft tissue comprises type I collagen creating fibroblasts.

5. The composition of any of the preceding claims, wherein said connective soft tissue is selected from the following:
- skin, including scalp,
- mucosa of the mouth, nose, eyes and secretory glands, and
- periodontal tissue, including gingiva and periodontal ligament.

6. The composition of any of the preceding claims, wherein said composition is a formulation selected from the following: film, liquid, aerosol, suspension, solution, tincture, mask, serum, cream, paste, lotion, ointment, gel, balsam, foam.

7. The composition of any of the preceding claims, wherein said composition aids in cleaning and/or washing the subject's skin and/or oral cavity.

8. The composition of any of the preceding claims, wherein said subject is a human or animal.

9. The composition of any of the preceding claims for use as a medicament.

10. The composition of any of claims 1-8 for use in the treatment or prevention of a disease in said subject, wherein said subject is suffering from or is at risk of a disease correlated with a type I collagen breakdown or type I collagen shortage, and wherein said disease is selected from the following: atopic or seborrheic dermatitis, xerosis, eczema, psoriasis, acne, dry skin, cracks and skin ruptures, vitiligo, alopecia, skin carcinogenesis, oral cancer, gingivitis, periodontitis, periodontal pockets, bleeding gum.

11. The composition of any of claims 1-8 for promoting fibroblast-mediated type I collagen synthesis in said connective soft tissue of said subject, and/or for preventing type I collagen destruction or shortage in said connective soft tissue of said subject.

12. A method of topical treatment of the skin and/or oral cavity of a living subject, wherein said method comprises a topical application of said skin and/or oral cavity with a composition of any of claims 1-8.

13. The method of claim 12, wherein said treatment comprises a topical application of said skin and/or oral cavity with said composition in a leave-on manner.

14. The use of the composition of any of the claims 1-8 for non-medical and/or aesthetic use, wherein said use is selected from skin cleaning, tooth and/or oral cavity cleaning or brushing.

15. The use of paeoniflorin in the manufacture of
- the composition of any of claims 1-8,
- the composition for use of claim 9,
- the composition for use of claim 10.
- the composition for promoting fibroblast-mediated type I collagen synthesis and/or for preventing type I collagen destruction or shortage of claim 11.
